# EUROPÄISCHE PATENTSCHRIFT

(11) **EP 0 761 653 B1**
(45) Veröffentlichungstag und Bekanntmachung des Hinweises auf die Patenterteilung: **20.05.1998**
(21) Anmeldenummer: 96112999.6
(22) Anmeldetag: 13.08.1996
(51) Int. Cl.: C07D 239/30

(54) **Verfahren zur Herstellung von 4,6-Dichlorpyrimidinen**
Process for the production of 4,6-dichloropyrimidine
Procédé pour la préparation de 4,6-dichloropyrimidine

(30) Priorität: 25.08.1995 DE 19531299
(43) Veröffentlichungstag der Anmeldung: 12.03.1997
(73) Patentinhaber: BAYER AG, 51368 Leverkusen (DE)
(72) Erfinder: Cramm, Günther, Dr., 51381 Leverkusen (DE); Käss, Volker, Dr., 51375 Leverkusen (DE); Steffan, Guido, Dr., 51519 Odenthal (DE)

(56) Entgegenhaltungen:
- EP-A- 0 251 246
- EP-A- 0 697 406
- WO-A-95/29166
- WO-A-96/23776
- BULL.SOC.CHIM.FRANCE, 1959, Seiten 741-742, XP002019799 HENNART ET AL: "Contribution à la synthèse de la dichloro-4-6 pyrimidine."
- J.CHEM.SOC., 1951, Seite 2214 XP002019800 HULL: "A New Synthesis of 4:6-Dihydroxypyrimidines"

## Beschreibung

Die vorliegende Erfindung betrifft ein verbessertes Verfahren zur Herstellung von 4,6-Dichlorpyrimidinen aus 4,6-Dihydroxypyrimidinen.

4,6-Dichlorpyrimidine sind Zwischenprodukte z.B. zur Herstellung von Pflanzenschutzmitteln und Farbstoffen.

Bei bekannten Verfahren zur Herstellung von 4,6-Dichlorpyrimidinen werden 4,6-Dihydroxypyrimidine mit Phosphoroxychlorid und einer Base wie Dimethylanilin oder Pyridin versetzt (s. J. Chem. Soc. 1943, 574; J. Chem. Soc. 1951, 2214; Bull. Soc. Chim. France 1959, 741 und Khim.-Pharm. Zhurnal 8 (12), 28 (1974) - engl. Übersetzung S. 741).

Zur Aufarbeitung wird dabei zunächst überschüssiges Phosphoroxychlorid abgezogen und dann der Rückstand entweder auf Eis ausgetragen und das Produkt durch Extraktion und Kristallisation gewonnen oder einer Sublimation unterworfen, bei der das Produkt als Sublimat erhalten wird. Nachteilig bei diesem Verfahren ist, daß die Basen in großen Mengen eingesetzt werden, sie aber nur mit erheblichem Aufwand zurückgewonnen und wiederverwendet werden können. Schließlich ist eine wäßrige Aufarbeitung sehr aufwendig wegen der Entsorgung der gebildeten Abwässer und der Handhabung von Extraktionsmitteln. Eine Aufarbeitung durch Sublimation ist in technischem Maßstab ebenfalls sehr aufwendig, z.B. im Hinblick auf die einzusetzenden Apparate und die arbeitshygienischen Erfordernisse, um das Produkt aus dem Sublimator zu entfernen.

Eigene Versuche, die bekannten Verfahren durch den Einsatz geringerer Mengen an Basen vorteilhafter zu gestalten scheiterten, weil dann die Ausbeute von 4,6-Dichlorpyrimidinen stark zurückgeht und die Bildung von Hochsiedern und Harzen stark zunimmt (siehe Beispiel 5).

Es wurde nun ein Verfahren zur Herstellung von 4,6-Dichlorpyrimidinen durch Umsetzung von 4,6-Dihydroxypyrimidinen mit überschüssigem Phosphoroxychlorid gefunden, das dadurch gekennzeichnet ist, daß man keine Base zufügt, nach der Umsetzung pro Äquivalent ausgetauschte Hydroxygruppe in Gegenwart von Phosphortrichlorid 0,7 bis 1,3 Mole Chlor so zufügt, daß stets ein Überschuß von Phosphortrichlorid gegenüber Chlor vorliegt und abschließend Phosphortrichlorid und Phosphoroxychlorid abtrennt.

In das erfindungsgemäße Verfahren kann man z.B. 4,6-Dihydroxypyrimidin oder in 2- und/oder 5-Stellung mit z.B C₁-C₁₀-Alkyl- und/oder C₆-C₁₀-Arylgruppen substituierte 4,6-Dihydroxypyrimidine einsetzen. Die Alkyl- und Arylgruppen können ihrerseits gegebenenfalls z.B. Halogen-, Nitro- und/oder C₁-C₆-Alkoxy als Substituenten enthalten. Unabhängig von einer Substitution in 2-Stellung können auch 5-halogen-, z.B. 5-chlorsubstituirte 4,6-Dihydroxypyrimidine zum Einsatz gelangen. Vorzugsweise setzt man 4,6-Dihydroxypyrimidin ein. Wenn im folgenden auf 4,6-Dihydroxypyrimidin oder 4,6-Dihydroxypyrimidine Bezug genommen wird sind die substituierten Typen als mit umfaßt anzusehen.

Pro Mol eingesetztes 4,6-Dihydroxypyrimidin kann man z.B. 2,5 bis 12 Mol Phosphoroxychlorid einsetzen. Vorzugsweise beträgt diese Menge 3,5 bis 5 Mol.

Für die Umsetzung von 4,6-Dihydroxypyrimidinen mit Phosphoroxychlorid sind beispielsweise Temperaturen im Bereich 60 bis 110°C geeignet. Bevorzugt sind Temperaturen von 80 bis 100°C. Man kann dabei z.B. so verfahren, daß man Phosphoroxychlorid vorlegt und das 4,6-Dihydroxypyrimidin zudosiert. Auch andere Arbeitsweisen sind möglich.

Vorzugsweise nachdem sich das eingesetzte 4,6-Dihydroxypyrimidin im Phosphoroxychlorid gelöst hat, setzt man dem Reaktionsgemisch Phosphortrichlorid und die oben angegebene Menge Chlor So zu, daß stets ein Überschuß von Phosphortrichlorid gegenüber Chlor im Reaktionsgemisch vorliegt. Der Überschuß wird vorteilhafterweise so bemessen, daß im Reaktionsgemisch stets 0,3 bis 5 Gew.-%, vorzugsweise 1 bis 3 Gew.-% freies Phosphortrichlorid vorliegen. Man kann dabei so verfahren, daß man Phosphortrichlorid und Chlor gleichmäßig und mit einer kurzen Vorgabe von Phosphortrichlorid zudosiert.

Pro Äquivalent ausgetauschte Hydroxygruppen kann man z.B. 0,75 bis 1,5 Mole Phosphortrichlorid, unter Beachtung des steten Vorliegens eines Überschusses davon im Hinblick auf Chlor, einsetzen.

Die Zugabe von Phosphortrichlorid und Chlor kann z.B. bei 60 bis 110°C erfolgen. Bevorzugt sind Temperaturen von 80 bis 100°C. Nach beendeter Zugabe von Phosphortrichlorid und Chlor kann es, insbesondere bei diskontinuierlicher Fahrweise vorteilhaft sein, bei 60 bis 110°C noch einige Zeit nachzurühren, z.B. 10 Minuten bis 3 Stunden.

Nach Abschluß der Umsetzung mit Phosphortrichlorid und Chlor kann das Reaktionsgemisch z.B. destillativ aufgearbeitet werden.

Vorteilhaft - wegen der Verdünnungswirkung des im Ansatz vorhandenen Phosphoroxychlorids - kann auch nur ein Teil des Reaktionsgemisches zur Aufarbeitung abgezweigt werden und der Rest, gegebenenfalls nach Zugabe von Phosphoroxychlorid, so daß dessen Anteil im Reaktionsgemisch einen vorgewählten Wert nicht unterschreitet, mit frischem 4,6-Dihydroxypyrimidin versetzt und nach Abreaktion des letzteren dann wieder mit der entsprechenden Menge Phosphortrichlorid und Chlor umgesetzt werden. Diese Verfahrensweise kann beliebig oft wiederholt werden.

Die Zugabe von Phosphortrichlorid kann auch vor oder gleichzeitig mit der Zugabe des 4,6-Dihydroxypyrimidins erfolgen. So kann es für die praktische Durchführung von Vorteil sein, das 4,6-Dihydroxypyrimidin als Suspension in Phosphortrichlorid zuzusetzen. In diesem Fall kann auf die Zugabe von Phosphortrichlorid vor der Chlorzugabe verzichtet werden.

Es hat sich als besonders günstig erwiesen, diese Reaktionsfolge und die anschließende destillative Trennung kontinuierlich durchzuführen.

Die Abtrennung von Phosphortrichlorid und Phosphoroxychlorid kann, je nachdem ob man das erfindungsgemäße Verfahren diskontinuierlich, diskontinuierlich in Schüben oder kontinuierlich durchführt auf unterschiedliche Weise erfolgen.

Bei diskontinuierlicher Fahrweise kann man z.B. das gesamte Reaktionsgemisch einer Destillation, vorzugsweise bei vermindertem Druck, unterwerfen und nacheinander Phosphortrichlorid und Phosphoroxychlorid abtrennen.

Bei diskontinuierlicher Fahrweise in Schüben kann man z.B. so vorgehen, daß man in ein ausreagiertes Reaktionsgemisch zunächst erneut 4,6-Dihydroxypyrimidin und danach Phosphortrichlorid und Chlor auf die oben beschriebene Weise, in den oben beschriebenen Mengen und bei den oben beschriebenen Temperaturen zusetzt und reagieren läßt. Diese erneute Zugabe kann, je nach der Größe des verwendeten Reaktionsgefäßes, mehrmals wiederholt werden, beispielsweise bis zu 20 Mal. Man kann dem dann vorliegenden Reaktionsgemisch einen Teil entnehmen, vorzugsweise mit einer Bandbreite von ± 20 Gew.-% soviel, wie der Gewichtszunahme seit der ersten Zugabe von 4,6-Dihydroxypyrimidin entspricht. Dem Rückstand kann man dann wieder Phosphoroxychlorid, danach 4,6-Dihydroxypyrimidin und danach Phosphortrichlorid und Chlor wie oben beschrieben zusetzen und nach Abklingen der Reaktion wieder einen Teil des Reaktionsgemisches entnehmen, vorzugsweise mit einer Bandbreite von ± 20 Gew.-% soviel, wie der Gewichtszunahme seit der Zugabe von 4,6-Dihydroxypyrimidin entspricht. Diesen Zyklus kann man beliebig oft wiederholen, z.B. bis zu 50 Mal.

Die bei dieser Fahrweise abgetrennten Anteile des Reaktionsgemisches kann man z.B. einer Destillation, vorzugsweise unter vermindertem Druck, unterwerfen und nacheinander Phosphortrichlorid und Phosphoroxychlorid abtrennen. Diese beiden Produkte können in das Verfahren recyclisiert werden. Überschüssiges Phosphoroxychlorid kann auf beliebige Weise weiterverwendet werden. Dies gilt auch für bei anderen Arbeitsweisen abgetrenntes Phosphortrichlorid und Phosphoroxychlorid.

Bei kontinuierlicher Fahrweise kann man z.B. das ausreagierte Reaktionsgemisch eines diskontinuierlichen Ansatzes einem Reaktionsgefäß zuführen, das eine Reaktionsführung mit minimaler oder keiner Rückvermischung gestattet, z.B. einem Mehrkammerreaktor, und dort kontinuierlich 4,6-Dihydroxypyrimidin, z.B. suspendiert in Phosphortrichlorid, und Phorphoroxichlorid unter Einhaltung der oben beschriebenen Bedingungen zusetzen. Der Reaktor, im Falle eines Mehrkammerreaktors z.B. einer mit 4 bis 12 Kammern, wird auf Reaktionstemperatur gehalten und Chlor in der oben beschriebenen Weise und in den oben beschriebenen Mengen räumlich entfernt von den anderen Zugabestellen eingespeist. Das den Reaktor verlassende Gemisch kann in einem Puffertank aufgefangen werden, von wo aus der Haupteil zurück in die Reaktion und der Rest in eine destillative Aufarbeitung geführt werden kann. Die destillative Aufarbeitung kann bei vermindertem Druck durchgeführt werden und beispielsweise dreiteilig angelegt sein, wobei man in einer ersten Destilliervorrichtung, z.B. einer Kolonne, überschüssiges Phosphortrichlorid, in einer zweiten Destilliervorrichtung, z.B. einer Kolonne, Phosphoroxychlorid und in einer dritten Destilliervorrichtung, z.B. einem Dünnschichtverdampfer, das hergestellte 4,6-Dichlorpyrimidin abtrennt.

Bei der diskontinuierlichen und der diskontinuierlichen Arbeitsweise in Schüben kann man aus den nach der Abtrennung von Phosphortrichlorid und Phosphoroxychlorid verbleibenden Rückständen die hergestellten 4,6-Dichlorpyrimidine durch Destillation, vorzugsweise unter vermindertem Druck, gewinnen.

Es ist vorteilhaft, bei der destillativen Abtrennung der hergestellten 4,6-Dichlorpyrimidine eine Schiebeflüssigkeit und/oder ein Fließmittel für den Destillationsrückstand einzusetzen. Geeignet sind hierfür beispielsweise hochsiedende, thermisch stabile Substanzen, z.B. Polywachse, etwa auf der Basis von Oligo- oder Polyethylenglykolen, Ditolylether, Polychlorbenzole und -toluole und Phthalsäuredialkylester.

Das erfindungsgemäße Verfahren gestattet die Herstellung von 4,6-Dichlorpyrimidinen in Ausbeuten von über 80 %, häufig über 85 % der Theorie. Dies ist besonders überraschend, da keine Base eingesetzt wird, was bisher für unabdingbar galt. Dadurch entfällt die Handhabung der Base und deren Abtrennung aus dem Reaktionsgemisch, was die Durchführung der Reaktion ganz wesentlich vereinfacht und auch eine kontinuierliche Arbeitsweise möglich macht.

Man kann das erfindungsgemäße Verfahren auch so betreiben, daß man 4,6-Dichlorpyrimidine in Ausbeuten von über 95 % der Theorie erhält. Man kann dies bei kontinuierlicher Arbeitsweise z.B. erreichen, indem man die kontinuierlich zugefügte Menge an 4,6-Dihydroxypyrimidinen reduziert und/oder den Anteil von Phosphoroxychlorid im Reaktionsgemisch erhöht und/oder die rückgeführte oder umlaufenden Menge an ausreagiertem Reaktionsgemisch erhöht. Derartige Steigerungen der chemischen Ausbeute gehen allerdings zu Lasten der Raum-Zeit-Ausbeute.

### Beispiele

### Beispiel 1

### a) Startphase:

Zu 2500 g Phosphoroxychlorid wurden bei 85 bis 90°C 28 g 4,6-Di-hydroxypyrimidin (im folgenden DHP bezeichnet) eingetragen. Nachdem dieses im Verlauf von 25 Minuten vollständig in Lösung gegangen war wurden 75 g Phosphortrichlorid zugesetzt und anschließend innerhalb von 15 Minuten 35 g Chlorgas zwischen 85 und 90°C eingeleitet. Nach beendeter Gasentwicklung wurden erneut 28 g DHP zugesetzt und wiederum, nachdem es in Lösung gegangen war, das Reaktionsgemisch wie zuvor mit 75 g Phosphortrichlorid und 35 g Chlor versetzt. Nach 10 Zyklen wurde dem Reaktionsgemisch soviel entnommen, wie der Gewichtszunahme entsprach (1.175 g).

Nach dieser Entnahme wurden dem Reaktionsansatz zunächst 250 g Phosphoroxychlorid zugesetzt, so die ursprüngliche Startmenge von 2.500 g Phosphoroxychlorid wieder hergestellt, danach 5 weitere Zyklen durchgeführt und dann 825 g Reaktionsgemisch entnommen.

Dieser Vorgang wurde noch zweimal wiederholt, wobei zunächst 335 g Phosphoroxychlorid zugesetzt und nach 5 Zyklen 910 g Reaktionsgemisch entnommen wurden. Nach Zugabe weiterer 400 g Phosphoroxychlorid - nochmals 5 Zyklen - und Entnahme von 970 g Reaktionsgemisch war die Startphase abgeschlossen.

### b) Dauerphase:

Im folgenden wurden jeweils 425 g Phosphoroxychlorid zugesetzt, jeweils 5 Zyklen durchgeführt und danach konstant 1.000 g Reaktionsgemisch entnommen. Das entnommene Reaktionsgemisch wurde destillativ aufgetrennt. Dabei wurde zunächst bei 100 mbar ein geringer Vorlauf von Phosphortrichlorid erhalten, anschließend bei 100 mbar und einer Sumpftemperatur von max. 130°C das Phosphoroxychlorid isoliert und zum Abschluß bei bis zu 20 mbar erniedrigtem Druck und einer Sumpftemperatur von max. 175°C das hergestellte 4,6-Dichlorpyrimidin abgetrennt. Es verblieb ein Destillationsrückstand von 12 Gew.-%, bezogen auf das erhaltene 4,6-Dichlorpyrimidin.

Die destillative Auftrennung der 1.000 g Reaktionsgemisch aus der Dauerphase ergab folgende Durchschnittswerte:
20 g Phosphortrichlorid,
800 g Phosphoroxychlorid,
160 g 4,6-Dichlorpyrimidin und
20 g Rückstand.

Bezogen auf die insgesamt eingesetzten 140 g DHP entspricht das einer Durchschnitts-Ausbeute an 4,6-Dichlorpyrimidin von 85,9 % der Theorie.

### Beispiel 2

Es wurde verfahren wie in Beispiel 1, jedoch wurde nach der Durchführung der ersten 10 Zyklen und der Entnahme des bis dahin angefallenen Überschusses vor jedem weiteren Zyklus 80 g Phosphoroxychlorid zugesetzt und die Zuwachsmenge an Reaktionsgemisch (200 g) nach jedem Zyklus entnommen und aufgearbeitet. Die Ergebnisse waren mit denen des Beispiels 1 vergleichbar.

### Beispiel 3

Es wurde verfahren wie in Beispiel 1, jedoch wurden die 28 g DHP nicht als Feststoff, sondern suspendiert in 75 g Phosphortrichlorid dem Ansatz zugesetzt und dafür kein Phosphortrichlorid vor der Chloreinspeisung zugegeben. Die Ergebnisse waren mit denen des Beispiels 1 vergleichbar.

### Beispiel 4

In einem 200 l fassenden Anmaischkessel wurden stündlich kontinuierlich 28 kg DHP mit 75 kg Phosphortrichlorid versetzt und die erhaltene Suspension in den unteren Bereich eines Mehrkammerreaktors gepumpt, dessen Inhalt auf 85 bis 90°C temperiert wurde. Der Mehrkammerreaktor bestand aus 8 Kammern und besaß ein Gesamtvolumen von 2 m³. Gleichzeitig wurden über zwei weitere Einspeisestutzen im unteren Bereich des Mehrkammerreaktos stündlich 80 kg Phosphoroxychlorid und 3 160 kg ausreagiertes Reaktionsgemisch aus einem Pufferbehälter zudosiert. Über einem Einspeisestutzen im mittleren Bereich des Mehrkammerreaktors wurden kontinuierlich 35 kg Chlor pro Stunde gasförmig eingespeist.

Der Überlauf des Mehrkammerreaktors wurde in einem Puffertank aufgefangen. Von hier wurde das Reaktionsgemisch zu einem Teil in den Mehrkammerreaktor zurückgefahren und der Rest (200 kg pro Stunde) einer destillativen Aufarbeitung zugeführt. Dort wurde in einer ersten Kolonne überschüssiges Phosphortrichlorid, in einer zweiten Kolonne Phosphoroxychlorid und nach Zugabe von Polyethylenglykol das hergestellte 4,6-Dichlorpyrimidin in einem Dünnschichter bei 100 mbar abgetrennt. Stündlich wurden 167 kg 4,6-Dichlorpyrimidin erhalten, das entspricht einer Ausbeute an 4,6-Dichlorpyrimidin von 89,8 % der Theorie.

### Beispiel 5 (zum Vergleich)

460 g Phosphoroxychlorid und 62 g N,N-Dimethylanilin wurden gemischt und in das Gemisch bei 100°C im Verlauf von 5 Stunden 116 g DHP (98 gew.-%ig) mit einer Schnecke eindosiert. Danach wurde bei 106 bis 128°C 8 Stunden nachgerührt. Das Reaktionsgemisch wurde mit 300 g Chlorbenzol verdünnt und auf 1,2 kg Eis ausgetragen. Die organische Phase wurde abgetrennt und zweimal mit je 100 ml Wasser gewaschen und anschließend fraktioniert destilliert. So wurden 85,7 g 4,6-Dichlorpyrimidin (= 58 % der Theorie) erhalten.

## Patentansprüche

1. Verfahren zur Herstellung von 4,6-Dichlorpyrimidinen durch Umsetzung von 4,6-Dihydroxypyrimidinen mit überschüssigem Phosphoroxychlorid, dadurch gekennzeichnet, daß man keine Base zufügt, nach der Umsetzung in Gegenwart von Phosphortrichlorid pro Äquivalent ausgetauschte Hydroxygruppen 0,7 bis 1,3 Mole Chlor so zufügt, daß stets ein Überschuß von Phosphortrichlorid gegenüber Chlor vorliegt und abschließend Phosphortrichlorid und Phosphoroxychlorid abtrennt.

2. Verfahren nach Anspruch 1, dadurch gekennzeichnet, daß man 4,6-Di-hydroxypyrimidin oder in 2- und/oder 5-Stellung mit C₁-C₁₀-Alkyl- und/oder C₆-C₁₀-Arylgruppen oder in 5-Stellung mit Halogen substituierte 4,6-Dihydroxypyrimidine einsetzt.

3. Verfahren nach Ansprüchen 1 und 2, dadurch gekennzeichnet, daß man pro Mol eingesetztes 4,6-Dihydroxypyrimidin 2,5 bis 12 Mol Phosphoroxychlorid einsetzt.

4. Verfahren nach Ansprüchen 1 bis 3, dadurch gekennzeichnet, daß man die Umsetzung von 4,6-Dihydroxypyrimidin mit Phosphoroxychlorid und die Zugabe von Phosphortrichlorid und Chlor bei Temperaturen von 60 bis 110°C durchführt.

5. Verfahren nach Ansprüchen 1 bis 4, dadurch gekennzeichnet, daß man es diskontinuierlich, diskontinuierlich in Schüben oder kontinuierlich durchführt.

6. Verfahren nach Ansprüchen 1 bis 5, dadurch gekennzeichnet, daß im Reaktionsgemisch stets 0,3 bis 5 Gew.-% freies Phosphortrichlorid vorliegen.

7. Verfahren nach Ansprüchen 1 bis 6, dadurch gekennzeichnet, daß man das hergestellte 4,6-Dichlorpyrimidin durch Destillation abtrennt.

8. Verfahren nach Ansprüchen 1 bis 7, dadurch gekennzeichnet, daß man das hergestellte 4,6-Dichlorpyrimidin durch Destillation unter Einsatz einer Schiebeflüssigkeit und/oder eines Fließmittels für den Destillationsrückstand abtrennt.

## Claims

1. Process for the preparation of 4,6-dichloropyrimidines by the reaction of 4,6-dihydroxypyrimidines with an excess of phosphoryl chloride, characterized in that no base is added, after the reaction 0.7 to 1.3 mol of chlorine are added per equivalent of exchanged hydroxyl groups in the presence of phosphorus trichloride so that an excess of phosphorus trichloride with respect to chlorine is always present, and finally phosphorus trichloride and phosphoryl chloride are removed.

2. Process according to Claim 1, characterized in that 4,6-dihydroxypyrimidine or 4,6-dihydroxypyrimidines substituted in position 2 and/or position 5 with C₁-C₁₀-alkyl and/or C₆-C₁₀-aryl groups or in position 5 with halogen are used.

3. Process according to Claims 1 and 2, characterized in that for each mole of 4,6-dihydroxypyrimidine used, 2.5 to 12 mol of phosphoryl chloride are used.

4. Process according to Claims 1 to 3, characterized in that the reaction of 4,6-dihydroxypyrimidine with phosphoryl chloride and the addition of phosphorus trichloride and chlorine are carried out at temperatures of 60 to 110°C.

5. Process according to Claims 1 to 4, characterized in that it is carried out batchwise, semicontinuously, or continuously.

6. Process according to Claims 1 to 5, characterized in that 0.3 to 5% by weight of free phosphorus trichloride is always present in the reaction mixture.

7. Process according to Claims 1 to 6, characterized in that the 4,6-dichloropyrimidine produced is separated off by distillation.

8. Process according to Claims 1 to 7, characterized in that the distillation of the 4,6-dichloropyrimidine produced is carried out with use of a driving liquid and/or a flow agent for the distillation residue.

## Revendications

1. Procédé pour la préparation de 4,6-dichloropyrimidines par mise en réaction de 4,6-dihydroxypyrimidines avec de l'oxychlorure de phosphore en excès, caractérisé en ce qu'on n'ajoute pas de base, après la réaction en présence de trichlorure de phosphore, par équivalent de groupes hydroxyle échangés, on ajoute de 0,7 à 1,3 mole de chlore de telle sorte qu'un excès de trichlorure de phosphore par rapport au chlore soit toujours présent, puis on sépare le trichlorure de phosphore et l'oxychlorure de phosphore.

2. Procédé selon la revendication 1, caractérisé en ce qu'on met en oeuvre une 4,6-dihydroxypyrimidine ou bien des 4,6-dihydioxypyrimidines portant un substituant alkyle en C₁-C₁₀ et/ou aryle en C₆-C₁₀ en position 2 et/ou en position 5, ou un substituant halogéno en position 5.

3. Procédé selon les revendications 1 et 2, caractérisé en ce qu'on met en oeuvre, par mole de la 4,6-dihydroxypyrimidine mise en oeuvre, de 2,5 à 12 moles d'oxychlorure de phosphore.

4. Procédé selon les revendications 1 à 3, caractérisé en ce qu'on effectue la mise en réaction de la 4,6-dihydroxypyrimidine avec de l'oxychlorure de phosphore et l'addition de trichlorure de phosphore et de chlore à des températures de 60 à 110°C.

5. Procédé selon les revendications 1 à 4, caractérisé en ce qu'on l'effectue en discontinu, en discontinu par lots ou en continu.

6. Procédé selon les revendications 1 à 5, caractérisé en ce que, dans le mélange réactionnel, du trichlorure de phosphore libre est toujours présent à concurrence de 0,3 à 5% en poids.

7. Procédé selon les revendications 1 à 6, caractérisé en ce qu'on sépare, par distillation, la 4,6-dichloropyrimidine préparée.

8. Procédé selon les revendications 1 à 7, caractérisé en ce qu'on sépare par distillation la 4,6-dichloropyrimidine préparée en mettant en oeuvre un liquide de déplacement et/ou un solvant pour le résidu de distillation.
